Europäisches Patentamt

European Patent Office

Office européen des brevets

(19)

(11) EP 0 603 675 B1

(12) EUROPÄISCHE PATENTSCHRIFT

(45) Veröffentlichungstag und Bekanntmachung des Hinweises auf die Patenterteilung:
12.11.1997 Patentblatt 1997/46

(51) Int Cl.⁶: **C08G 18/10**, C08G 18/08, C08G 18/73, C08G 18/75, A61L 27/00

(21) Anmeldenummer: 93119952.5

(22) Anmeldetag: 10.12.1993

(54) **Katalysatorfreie aliphatische thermoplastische Polyurethane**

Catalyst-free aliphatic thermoplastic polyurethanes

Polyuréthanes aliphatiques et thermoplastiques sans catalyseur

(84) Benannte Vertragsstaaten:
CH DE DK ES FR GB IT LI NL SE

(30) Priorität: 23.12.1992 DE 4243782
07.05.1993 DE 4315173

(43) Veröffentlichungstag der Anmeldung:
29.06.1994 Patentblatt 1994/26

(73) Patentinhaber: BAYER AG
51368 Leverkusen (DE)

(72) Erfinder:
• **Müller, Hanns-Peter, Dr.**
**D-51519 Odenthal (DE)**
• **Dhein, Rolf, Dr.**
**D-47800 Krefeld (DE)**
• **Hugl, Herbert, Dr.**
**D-51467 Bergisch Gladbach (DE)**
• **Pudleiner, Heinz, Dr.**
**D-47800 Krefeld (DE)**

(56) Entgegenhaltungen:
EP-A- 0 461 375         WO-A-92/04390
DE-A- 3 341 847         DE-A- 3 643 465
FR-A- 1 497 772         US-A- 4 024 113

Bemerkungen:
Die Akte enthält technische Angaben, die nach dem Eingang der Anmeldung eingereicht wurden und die nicht in dieser Patentschrift enthalten sind.

## Beschreibung

Die vorliegende Erfindung betrifft reine Polyether- und Polyesterurethane basierend auf aliphatischen und/oder cycloaliphatischen Diisocyanaten sowie die Verwendung dieser reinen Polyurethankunststoffe für biomedizinische Anwendungen. Thermoplastische Polyurethanelastomere (TPU) sind seit langem bekannt (Kunststoff Handbuch Bd. 7 (1983) ISBN 3-446-13614-2,S. 428 ff.).

Die Verwendung derartiger TPU für biomedizinische Anwendungen beschreibt z.B. Gogolewski in Colloid Polym. Sci 267 757-785 (1989), Keywords: Biomedical polyurethanes, chemistry, structure-property relations, tissue-material interaction, surface properties, biomedical use, compatibility properties.

Gogolewskii kommt auf S. 782 zu dem Schluß: "Biocompatible and blood compatible polyurethane elastomers with unique physical and mechanical properties resulting from the hard-segment-soft-segment microphase segregation, are materials of choice for a number of biomedical applications.

The two-step solution ploymerization process which leads to polyurethanes with better physical characteristics, is preferred to one-step solution or melt polymerizations.

The purity of reactants and polymerization media is critical to the final properties of polyurethanes.

The purity of biomedical polyurethanes (i.e., effective removal from the polymer of catalyst residues, low molecular weight fractions, processing aids, etc.) determine to a great extent their in vivo performance (biocompatibility, blood compatibility, molecular stability)."

"Research on polyurethanes should be continued to develop the "ultimate" biomedical polyurethanes of tomorrow."

In den letzten Jahren haben viele Autoren und Firmen sogenannte biokompatible Polyurethane zu Patenten angemeldet. So beschreibt z.B. G. G.Wick, Akzo gmbH in DE 3643465 (1986) ein Verfahren zur Herstellung von biokompatiblen Polyurethanen durch Umsetzung von cycloaliphatischen Diisocyanaten mit einem Makrodiol zu einem NCO-Gruppen aufweisenden Voraddukt, wobei man 3 bis 33 Mol Diisocyanat pro Mol Macrodiol einsetzt und Kettenverlängerung des Voraddukts mit einem Gemisch aus niedermolekularem aliphatischen Diol und einem aliphatischen und/oder cycloaliphatischen Macrodiol, dadurch gekennzeichnet, daß man in dem Gemisch der Kettenverlängerer als aliphatisches Diol Trimethylhexandiol verwendet. Der Zusatz von Zinnkatalysatoren ist bei den Ausführungsbeispielen vorgeschrieben.

Im EP EP 0461375 und US 5133742 wird ein thermoplastisches Polyurethan (TPU) beschrieben, das für medizinische Zwecke geeignet ist. Das bevorzugte, beschriebene TPU ist aufgebaut aus Polycarbonatdiol (MG 1898) MDI und 1,4-Butandiol.

In PCT WO 92/04390 beschreibt M. SZYCHER Polymedica Industries, Inc. mit einer Priorität von 12.09.90 US "Biostable Polyurethane Products". Die dort beschriebenen Polyurethane werden aufgebaut aus organischen Diisocyanaten, bevorzugt aliphatischen und/oder cycloaliphatischen Diisocyanaten mit Polycarbonatdiol, kettenverlängert mit Diol, Diamin oder einer Mischung aus Diamin und Alkanolamin. Die Verwendung von Zinnkatalysatoren belegt Beispiel 1.

E. Müller hat schon 1969 gefunden (vgl. Angew. Makromol. Chemie 14 (1970), 75-86), daß Polyurethanelastomere mit der höchsten Hydrolysebeständigkeit aus 1,6-Hexandiolpolycarbonat erhalten werden.

Gogolewski stellt fest, daß aliphatische Polyurethane für biomedizinische Zwecke den aromatischen Polyurethanen vorzuziehen sind.

Aliphatische bzw. cycloaliphatische Polyisocyanate reagieren jedoch mit Diolkomponenten zu langsam, so daß nach allen bekannten Verfahren des Standes der Technik den Reaktionsgemischen Katalysatoren zugesetzt werden müssen. Als Katalysatoren haben sich insbesondere Zinnoctoat, Dibutylzinndilaurat und/oder tert. Amine wie z.B. Diazabicyclooctan (DABCO) bewährt.

Aufgabe der vorliegenden Erfindung ist es daher, Polyurethane zur Verfügung zu stellen, die möglichst frei von Zusatzstoffen sind und insbesondere ohne den Zusatz von Katalysatoren hergestellt werden. Die Polyurethane sollen aus aliphatischen und/ oder cycloaliphatischen Diisocyanaten aufgebaut sein und dadurch reine, Polyurethane darstellen, die auch in der Medizintechnik einsetzbar sind.

Alle bekannten Verfahren des Standes der Technik, die zum Aufbau von TPU verwendet werden, arbeiten bei möglichst tiefen Temperaturen, um unerwünschte Nebenreaktionen zu vermeiden. Die wichtigsten Nebenreaktionen sind die Dimerisierung von Diisocyanaten, die Trimerisierung, die Carbodiimidbildung, die Allophanatbildung, die Biuretbildung.

Beim Aufbau von TPU muß insbesondere mit der Allophanatbildung unter Molekülverzweigung gerechnet werden. Kunststoffhandbuch Bd.7 Polyurethane (1983) enthält auf S. 82 den Hinweis, daß diese Reaktion auch unkatalysiert bei etwa 120°C bis 140°C durchgeführt werden kann.

Der Aufbau der TPU wird üblicherweise über NCO-Prepolymere vorgenommen. D. Dieterich in Houben-Weyl, Bd. E 20, S. 1613-1617, "Katalysatoren beeinflussen die Zusammensetzung der Produkte, während die Temperatur (unter 100°C), die Reaktionszeit und der Zugabemodus eine untergeordnete Rolle spielen."

Aus allen diesen genannten Veröffentlichungen geht hervor, daß zum Aufbau von TPU über NCO-Prepolymer und Semiprepolymere Temperaturen unter 100 °C bevorzugt werden.

Zur Herstellung transparenter, nicht vergilbender Elastomeren, insbesondere für biomedizinische Anwendungen, können 1,6-Bis-[isocyanat]-hexan(HDI) bzw. 5-Isocyanat-3-(isocyanat-methyl)- 1,3,3-trimethylcyclohexan (IPDI)-Prepolymere eingesetzt werden. Zur Glykol-Verlängerung muß stark katalysiert werden (D. Dieterich in Houben-Weyl., Bd. E 20, S. 1637 dort weitere Literaturangaben).

Aus diesen literaturbekannten Verfahren mußte der Fachmann den Schluß ziehen, daß aliphatische TPU ohne Katalysatoren nicht herstellbar sind.

Es ist aus den genannten Gründen völlig überraschend, daß gemäß der vorliegenden Erfindung besonders reine und mechanisch hochwertige aliphatische TPU hergestellt werden können, wenn man bei Temperaturen über 100°C ohne den Zusatz von Katalysatoren arbeitet.

Gegenstand der vorliegenden Erfindung sind daher:

Katalysatorfreie thermoplastische, Polyurethane, enthaltend aliphatische und/oder cycloaliphatische Diisocyanate, Macrodiole vom MG500 bis 10000 und Kettenverlängerer vom MG62 bis 400, erhältlich durch Umsetzung von aliphatischen und/oder cycloaliphatischen Diisocyanaten mit einem Macrodiol zu einem NCO-Gruppen aufweisenden Voraddukt, wobei man 1,5 bis 22 Mol Diisocyanat pro Mol Macrodiol einsetzt, und die Kettenverlängerung des Voraddukts mit niedermolekularen aliphatischen und/oder cycloaliphatischen Diolen vornimmt, gegebenenfalls auch weiteres Diisocyanat - unter Beibehaltung des obengenannten NCO/OH-Verhälmisses- als Kettenverlängerer zuführt, man die Voradduktbildung bei 90-150°C durchführt und die Kettenverlängerung bei Temperaturen von 90-230°C vornimmt.. mit der Maßgabe, daß man die genannten Reaktionsbedingungen mindestens so lange aufrecht erhält, bis eine Schmelzviskosität von mindestens 2500 mPas, gemessen bei 120°C, erreicht ist und anschließend bei erhöhter Temperatur von 130°C bis 240 °C die Polyaddition zu einem beliebigen Zeitpunkt zu Ende führt; sowie die Verwendung der reinen. kompatiblen Polyurethankunstsoffen zur Herstellung von Implantaten insbesondere zur Herstellung von Kathetern und Schläuchen, zur Herstellung von Blutbeuteln, medizinisch angewendeten Folien, Klebstoffen und beliebigen Formkörpern als orthopädische Materialien. Selbstverständlich sind derartige Polyurethane auch außerhalb der Medizintechnik in anderen Bereichen einsetzbar.

Die Aufgabe der Erfindung wird gelöst durch ein Verfahren zur Herstellung von gegebenenfalls in einem organischen Lösungsmittel gelösten, reinen biokompatiblen Polyurethanen durch Umsetzung von aliphatischen und/oder cycloaliphatischen Diisocyanaten mit einem Macrodiol zu einem NCO-Gruppen aufweisenden Voraddukt, wobei man 1,5 bis 22 Mol Diisocyanat pro Mol Macrodiol einsetzt und Kettenverlängerung des Voradduks mit geeigneten niedermolekularen Kettenverlängerern oder Kettenverlängerergemischen in den im folgenden näher beschriebenen Verfahrensweisen durchführt.

Zum Aufbau des Voraddukts (NCO-Semiprepolymer) werden bevorzugt Macrodiole vom MG 500 bis 2000 verwendet. Dabei handelt es sich um die bekannten Polyether auf Basis Tetrahydrofuran oder Ethylenoxid/Propylenoxid, Polyester z.B. auf Basis von Adipinsäure und Diolen bzw. Polycarbonate wie 1,6-Hexandiolpolycarbonat, Polyester auf Basis von ε-Caprolacton, cycloaliphatische Polycarbonate oder deren Mischungen, wie sie im Prinzip in der Zusammenfassung von Gogolewski beschrieben sind (s. 762 PEO, PTMO, PPO, PIB, PEA, PCL, PDMS-OH, PBD). Dabei können auch Polyisobutylene und/oder Polysiloxane mitverwendet werden.

Bevorzugte Diisocyanate sind die bekannten aliphatischen und cycloaliphatischen Diisocyanate 4,4'-Dicyclohexylmethan-diisocyanat ($H_{12}$MDI), trans-1,4-Cyclohexandiisocyanat (CDI), Isophorondiisocyanat (IPDI), Hexamethylendiisocyanat (HDI), 1,1,6,6-Tetrahydroperfluorohexamethylen-diisocyanat, Tetramethylxylylen-diisocyanat (TMXDI) oder Di-meryl-diisocyanat (DDI). Verwendet man $H_{12}$MDI, so können sowohl die technischen Gemische (20 % tt/50 % tC/30 % C,C) als auch die angereicherten Gemische mit höheren t/t (trans/trans) Gehalt verwendet werden. Reines t/t ist ebenfalls einsetzbar. Als Kettenverlängerer werden die bekannten, aktive Wasserstoffatome tragenden di- oder polyfuktionellen Verbindungen vom MG 62 bis 400 eingesetzt. Verwendet man trifunktionelle oder tetrafunktionelle Verbindungen, so entstehen vernetzte Polyurethane als Gießsysteme. Verwendet man difunktionelle Verbindungen, wie z.B. Ethylenglykol, Diethylenglykol, Dipropylenglykol, 1,4-Butandiol, 1,6-Hexandiol, Trimethylhexandiol, 1,8-Octandiol, Neopentylglykol oder 1,12-Dodecandiol, Cyclohexyl-dimethanol, 1,4-Cyclohexandiol oder Perhydrobisphenol-A, so entstehen thermoplastische Polyurethan-Kunststoffe. Trifunktionelle Vernetzer sind z.B Glycerin, Trimethylolpropan, höherfunktionelle Vernetzer sind z.B. Pentaerythrit oder Sorbit.

Sowohl die Umsetzung zum Voraddukt (Semiprepolymer) als auch die Kettenverlängerung können nach an sich in der Polyurethanchemie üblichen Verfahrensweisen durchgeführt werden. Das Macrodiol wird im Kessel vorgelegt und bei 120 °C unter Anlegen eines Vakuums entwässert. Durch Zugabe von 1,5 bis 22 Mol Diisocyanat pro Mol Macrodiol wird das Prepolymer oder das Semiprepolymer aufgebaut. Die Umsetzung wird im allgemeinen ohne Lösungsmittel durchgeführt.

Bei der Kettenverlängerung wird im allgemeinen mit einem geringen Überschuß an NCO-Gruppen pro OH-Gruppen des Kettenverlängerers gearbeitet. Ein Verhältnis von NCO-Gruppen zu OH-Gruppen im Gemisch von 1,15:1 bis 1,01:1 wird bevorzugt. Man kann aber auch mit äquivalenten Mengen oder mit OH-Gruppen-Überschuß arbeiten. Der Einsatz von monofunktionellen Kettenabbrechern ist ebenfalls möglich. Da bekannterweise die Kettenverlängerer mit den Diisocyanaten die Hartsegmente ausbilden, ist der Anteil an Diisocyanat in den Ausgangsstoffen von Bedeutung. So lassen sich durch den gewichtsmäßigen Anteil des Diisocyanats, mechanische Eigenschaften wie Härte, Erweichungsbereich, Fließ- und Schmelzbereich steuern. Erfindungswesentlich ist das Arbeiten ohne zusätzliche Katalysatoren bis zu einer Mindestviskosität. Durch diese Verfahrensweise wird in homogener Phase das Polymer ohne Nebenreaktionen aufgebaut. Man erhält völlig klare stippenfreie Polyurethankunststoffe. Bei kleinen Ansätzen wird das Semiprepolymer mit den Kettenverlängerern gemischt und auf 130 bis 180 °C, vorzugsweise 130-150 °C erwärmt. Dabei wird der Polymeraufbau über die NCO-Zahl und die Viskosität kontrolliert. Hat sich eine Viskosität von 2500 mPas, gemessen bei 120°C, aufgebaut, so gießt man den Ansatz aus und führt die Polyaddition im Heizschrank zu Ende.

In der Technik wird dagegen so verfahren, daß man Semiprepolymere und Kettenverlängerer über geeignete Mischaggregate mischt (Düsen, Mischkopf, Statikmischer), das erhaltene homogene Gemisch über einen heizbaren Rohrreaktor über geeignete Pumpen fördert, gegebenenfalls Kettenabbrecher zudosiert, und nach Aufbau der gewünschten Viskosität einer Schneckenmaschine zuführt (z.B. ZSK 32) und die Polyaddition im Temperaturbereich von 140 bis 250°C, bevorzugt 140 bis 240°C zu Ende führt. Aus dem Extruder werden homogene, stippenfreie Polymerstränge extrudiert, die nach Abkühlen granuliert werden. Das Granulat kann vor der weiteren Verwendung in Silos nachgetempert werden.

Technisch läßt sich die Reaktivextrusion ebensogut anwenden wie das sogenannte Band- oder Band-Extruder-Verfahren. Bei diesen Verfahren wird die Reaktionsmasse auf einem Band abgelegt. Auf diesem Band durchläuft das Material verschiedene Temperaturzonen, in denen die Reaktion vervollständigt wird.

In einer weiteren erfindungsgemäßen Verfahrensweise geht man so vor, daß man in einem geeigneten Kessel das Makrodiol vorlegt und unter Rühren und Vakuum bei 120°C entwässert. Danach fügt man unter Stickstoff die gesamte Menge an aliphatischem und/oder cycloaliphatischem Diisocyanat zu und erhitzt den Kessel auf 150°C. Nach ca. 1 Stunde hat sich dann das NCO (Semi)-prepolymer gebildet, was durch Titration des NCO-Gehaltes kontrollierbar ist. Danach dosiert man unter kontinuierlichem Aufheizen dem Kessel die berechnete Menge an Diol-Kettenverlängerer zu und rührt die entstandene Schmelze des TPU noch 1 Stunde bei 210-240°C nach. Die hochviskose, gut rührbare Schmelze wird anschließend auf Teflonbleche abgelassen. Man erhält auf diese Weise ein bei Raumtemperatur klebfreies, klares, farbloses Polyurethan.

Diese Verfahrensweise ist immer dann anzuwenden, wenn bei Kennzahlen > 100 gearbeitet wird.

Will man dagegen TPU mit Kennzahlen < 100 synthetisieren, so ist bei der Kesselfahrweise folgender segmentierter Aufbau vorzunehmen. Vorlage des Makrodiols und Entwässerung wie oben beschrieben. Anschließend dosiert man auf die berechnete Menge Makrodiol 1,5-3 Mol Diisocyanat zu und läßt zum NCO-Prepolymer abreagieren (bis 150°C). Nach der NCO-Kontrolle fügt man dem Ansatz die gesamte berechnete Menge des Diol-Kettenverlängerers zu. Nach Abklingen der exothermen Reaktion dosiert man bei 150-240°C unter Zuführen von Wärme die berechnete Menge an Diisocyanat hinzu, rührt die Schmelze des TPU noch 1 Stunde nach und läßt die Schmelze auf ein Kühlband fließen (z.B. Wasserbad, oder gekühltes Kaiserband)). Auf diese Weise werden TPU mit Kennzahlen < 100 synthetisiert.

Eine weitere Variation dieses erfindungsgemäßen Verfahrens besteht darin, die Prepolymer-Synthese aus dem Makrodiol und dem verwendeten Diisocyanat in einem Molverhältnis von 1 Mol Makrodiol/1,5 Mol Diisocyanat zu bilden. Dabei bilden sich NCO-Prepolymere, die über Diisocyanate vorverlängerte Weichsegmente besitzen. Die anschließende Verfahrensweise kann, gegebenenfalls nach weiterer Diisocyanatzugabe, nach beiden beschriebenen Verfahren (Kz > 100, KZ < 100) durchgeführt werden.

Besonders vorteilhaft ist, daß man nach dem Verfahren der vorliegenden Erfindung reine, biokompatible Polyurethane erhält. Die Mitverwendung von Gleitmitteln für die bessere Verarbeitung ist möglich. Dabei wird bevorzugt das Stearylamid des Ethylendiamins verwendet (®Hoechstwachs C, EBS-Wachs Rhenax 12).

Es versteht sich von selbst, daß insbesondere bei Polyurethanen, die als Formkörper für Implantationszwecke verwendet werden sollen, hochreine Ausgangssubstanzen eingesetzt werden. Dies gilt insbesondere für die eingesetzten Diisocyanate und niedermolekularen Kettenverlängerer.

Die erfindungsgemäßen Polyurethane können nach üblichen Verfahren, wie z.B. Extrudieren, Spritzgießen, Schlauchextrusion, Folienextrusion, zu Formkörpern verarbeitet werden. Die Formkörper insbesondere die Schläuche sind knickfest, klebfrei, durchsichtig, stippenfrei, weich oder steif, flexibel und insbesondere sterilisierbar (Heißdampf, EO, $\gamma$-Bestrahlung).

Es ist auch möglich, das Polyurethan in Lösung zu bringen und dann aus der Lösung zu Verarbeiten (Tauchen, Gießen, Koagulationsverfahren). Als Lösungsmittel seien genannt Chloroform, Dimethylformamid, Dimethylacetamid, N-Methylpyrrolidon oder Toluol/Isopropanol-Mischungen.

Besonders vorteilhaft ist es, daß man, ohne dabei die hervorragenden mechanischen Eigenschaften sowie ihre Biokompatibilität zu verschlechtern, durch geringfügiges Variieren der Einstellung des Verhältnisses NCO-Gruppen zu OH-Gruppen die Oberflächenbeschaffenheit der aus diesen Polyurethanen hergestellten Formkörper in chemischer Hinsicht variieren kann. So ist es möglich, durch Steuerung des Isocyanatüberschusses die Reaktionsfähigkeit des Polyurethans mit anderen, mit Isocyanat reagierenden Gruppen zu verändern. Dadurch ist es möglich, die Kompatibilität und den Grad der Verbindung der Polyurethankörper mit der Umgebung zu beeinflussen. So kann man durch die Anzahl der noch reaktionsfähigen Isocyanatgruppen unterschiedliche Wechselwirkungen zwischen dem Polyurethanformkörper und der Umgebung, in welche der biokompatible Formkörper implantiert wird, ausüben.

Die reinen, insbesondere zinnfreien biokompatiblen Polyurethane sind für Anwendungen sowohl in der Veterinär- als auch der Humanmedizin, aber auch in anderen gebieten der Technik, wie z.B. als lichtechte Folien für Autoinnenausstattungen, Verbundgläser, Formkörper, Spritzgußartikel, Dichtungsmaterialien, Verbundwerkstoffe oder Verpackungsmaterial geeignet.

Die Erfindung wird durch die nachfolgenden Beispiele näher erläutert.

Als Ausgangsmaterialien werden eingesetzt:

®Desmophen 2020 (Bayer AG)
1,6-Hexandiol-polycarbonat MG 2000. OH-Zahl 56

®Desmodur W (Bayer AG) $H_{12}MDI$
4,4'-Dicyclohexylmethan-diisocyanat

®Terathane 1000 (DuPont)
Polytetrahydrofuran MG 1000, OH-Zahl 112

Polyether L 1515, ein auf Propylenglykol gestarteter Polyether mit 87 % PO und 13 % EO, MG 750, OH-Zahl 150

Polyether L 5050, ein auf Propylenglykol gestarteter Polyether mit 50 % PO und 50 % EO als Mischblock, MG 2000, OH-Zahl 56

Polywachs 56, ein auf Diethylenglykol gestarteter Polyether mit 100 % EO, MG 2000, OH-Zahl 56

Der Hartsegmentanteil wurde aus der Summe aus Diisocyanat und kurzkettigem Diol berechnet (siehe auch Houben-Weyl, Band E 20, S. 1568ff.). Unter Kennzahl versteht man:

$$KZ = \frac{Mol\ NCO}{Mol\ OH} \times 100\ \text{(vergleiche auch S.6,Z. 11 ff.)}$$

**Beispiel 1:**

Zur Herstellung des isocyanathaltigen Prepolymeren werden im 2 l-Schliffbecherglas 800 g (0,4 Mol) Desmophen 2020 unter Rühren als Schmelze bei 120°C und 30 mbar entwässert. Die Temperatur wird auf 80°C gesenkt und 927 g (3,54 Mol) DesmodurW zugegeben. Zur Prepolymerbildung wird 5 h bei 110°C und 20 mbar gerührt. Dem Ansatz wird eine Probe zur NCO-Bestimmung entnommen. Der NCO-Gehalt beträgt 7,9 % NCO (ber. 7,95 %).

Nun wird zur Kettenverlängerung die Temperatur auf 90°C gesenkt und 20g Höchstwachs C sowie 274 g (3,04 Mol) Butandiol (1,4) zugegeben. Dann läßt man die Temperatur langsam (ca. 45 min) auf 130°C ansteigen und gießt den Ansatz auf ein Teflonblech aus. Die erneute NCO-Bestimmung nach dem Ausgießen ergibt einen NCO-Wert von 1,8 % NCO. Zur Vervollständigung der Reaktion wird 15 h bei 130°C getempert.

Das so erhaltene Plattenmaterial wird in Stücke geschnitten und zu Granulat vermahlen und für die Konfektionierung als Granulat im Extruder bei 190 °C und 20 bar homogenisiert. Das so erhaltene Material enthält 40% Weichsegment und 60 % Hartsegment, die Verarbeitungstemperatur für den Spritzguß beträgt 200 °C.

**Beispiel 2:**

Entsprechend Beispiel 1 werden aus 1000 g (0,5 Mol) Desmophen 2020 und 790 g (3,02 Mol) Desmodur W sowie 218 g (2,42 Mol) Butandiol-(1,4) ein TPU mit 50 % Weichsegment und 50 % Hartsegment erhalten.

**Beispiel 3:**

Entsprechend Beispiel 1 werden aus 1100 g (0,55 Mol) Desmophen 2020 und 721 g (2,75 Mol) Desmodur W sowie 189 g (2,1 Mol) Butandiol-(1,4) ein TPU mit 55 % Weichsegment und 45 % Hartsegment erhalten.

**Beispiel 4:**

Entsprechend Beispiel 1 werden aus 1200 g (0,6 Mol) Desmophen 2020 und 645 g (2,46 Mol) Desmodur W sowie 161 g (1,79 Mol) Butandiol-(1,4) ein TPU mit 60 % Weichsegment und 40 % Hartsegment erhalten.

**Beispiel 5:**

Entsprechend Beispiel 1 werden aus 1300 g (0,65 Mol) Desmophen 2020 und 570 g (2,17 Mol) Desmodur W sowie 132 g (1,47 Mol) Butandiol-(1,4) ein TPU mit 65 % Weichsegment und 35 % Hartsegment erhalten.

**Beispiel 6:**

Entsprechend Beispiel 1 werden aus 800 g (0,4 Mol) Desmophen 2020 und 867 g (3,31 Mol) Desmodur W sowie 333 g (2,82 Mol) Hexandiol-(1,6) ein TPU mit 40 % Weichsegment und 60 % Hartsegment erhalten.

**Beispiel 7:**

Entsprechend Beispiel 1 werden aus 1000 g (0,5 Mol) Desmophen 2020 und 736 g (2,81 Mol) Desmodur W sowie 264 g (2,24 Mol) Hexandiol-(1,6) ein TPU mit 50 % Weichsegment und 50 % Hartsegment erhalten.

**Beispiel 8:**

Entsprechend Beispiel 1 werden aus 1100 g (0,55 Mol) Desmophen 2020 und 675 g (2,57 Mol) Desmodur W sowie 230 g (1,95 Mol) Hexandiol-(1,6) ein TPU mit 55 % Weichsegment und 45 % Hartsegment erhalten.

## Beispiel 9:

Entsprechend Beispiel 1 werden aus 1200 g (0,6 Mol) Desmophen 2020 und 605 g (2,31 Mol) Desmodur W sowie 196 g (1,66 Mol) Hexandiol-(1.6) ein TPU mit 60 % Weichsegment und 40 % Hartsegment erhalten.

## Beispiel 10:

Entsprechend Beispiel 1 werden aus 800 g (0,4 Mol) Desmophen 2020 und 822 g (3,7 Mol) Isophorondiisocyanat sowie 378 g (3,2 Mol) Hexandiol-(1,6) ein TPU mit 40 % Weichsegment und 60 % Hartsegment erhalten.

## Beispiel 11:

Entsprechend Beispiel 1 werden aus 1000 g (0,5 Mol) Desmophen 2020 und 698 g (3,15 Mol) Isophorondiisocyanat sowie 302 g (2,56 Mol) Hexandiol-(1,6) ein TPU mit 50 % Weichsegment und 50 % Hartsegment erhalten.

## Beispiel 12:

Entsprechend Beispiel 1 werden aus 1200 g (0,6 Mol) Desmophen 2020 und 578 g (2,6 Mol) Isophorondiisocyanat sowie 227 g (1,92 Mol) Hexandiol-(1,6) ein TPU mit 60 % Weichsegment und 40 % Hartsegment erhalten.

## Beispiel 13:

Entsprechend Beispiel 1 werden aus 900 g (0,9 Mol) Terathane 1000 und 884 g (3,38 Mol) Desmodur W sowie 216 g (2,4 Mol) Butandiol-(1,4) ein TPU mit 45 % Weichsegment und 55 % Hartsegment erhalten.

## Beispiel 14:

Entsprechend Beispiel 1 werden aus 1000 g (1 Mol) Terathane 1000 und 816 g (3,11 Mol) Desmodur W sowie 185 g (2,05 Mol) Butandiol-(1,4) ein TPU mit 50 % Weichsegment und 50 % Hartsegment erhalten.

## Beispiel 15:

Entsprechend Beispiel 1 werden aus 800 g (0,8 Mol) Terathane 1000 und 945 g (3,61 Mol) Desmodur W sowie 243 g (2,7 Mol) Butandiol-(1,4) ein TPU mit 40 % Weichsegment und 60 % Hartsegment erhalten.

## Beispiel 16:

Entsprechend Beispiel 1 werden aus 800 g (1,07 Mol) Polyether L 1515 und 974 g (3,72 Mol) Desmodur W sowie 226 g (2,52 Mol) Butandiol-(1,4) ein TPU mit 40 % Weichsegment und 60 % Hartsegment erhalten.

## Beispiel 17:

Entsprechend Beispiel 1 werden aus 800 g (0,4 Mol) Polyether L 5050 und 925 g (3,54 Mol) Desmodur W sowie 274 g (3,04 Mol) Butandiol-(1,4) ein TPU mit 40 % Weichsegment und 60 % Hartsegment erhalten.

## Beispiel 18:

Entsprechend Beispiel 1 werden aus 800 g (0,4 Mol) Polywachs 56 und 925g (3,54 Mol) Desmodur W sowie 274 g (3,04 Mol) Butandiol-(1,4) ein TPU mit 40 % Weichsegment und 60 % Hartsegment erhalten.

## Beispiel 19:

(Vergleichsbeispiel, Nachstellung des Beispiels V aus PCT WO 92/04390) 1000 g (0,5 Mol) Hexandiolpolycarbonat (Desmophen 2020 BayerAG) werden bei 110°C und 20 mbar unter Rühren 1 h entwässert. Dann wird bei 80 °C 275 g (1,05 Mol) Desmodur W ($H_{12}$MDI) und 0,1 ml DBTL (Dibutylzinndilaurat) zugegeben und weitere 3 h bei 110 °C und 20 mbar gerührt. Anschließend wird die Temperatur auf 80 °C abgesenkt und zur Kettenverlängerung 59 g (0,5 Mol) 1,6-Hexandiol zugegeben. Infolge der sofort einsetzenden Verfestigung des Materials wird der Ansatz sofort auf ein Teflonblech mit einem Spatel verteilt und im Heizschrank bei 110 °C 5h getempert. Es entsteht ein weiches Polymer, das unter dem Mikroskop bis 300 °C nicht schmilzt, ab 300°C Zersetzungsreaktionen aufweist. Das Polymer ist in Methylenchlorid, DMF, DMAC oder N-Methyl-pyrrolidon nicht löslich.

## Beispiel 20:

(Nachstellung des Beispiels 19 unter den Bedingungen der vorliegenden Erfindung) Man arbeitet wie im Beispiel 19 beschreiben nur mit dem Unterschied, daß kein Zinnkatalysator verwendet und die Kettenverlängerungsreaktion unter den Bedingungen des Beispiels 1 durchgeführt wurde. Nach Tempern bei 130 °C erhält man ein Material, das in der Wärme noch Fäden zieht, bei Raumtemperatur aber fest, klebfrei und sehr weich ist. Dieses Polyurethan ist in Methylenchlorid, DMF, DMAC oder NMP löslich. Das so erhaltene Material enthält 75 % Weichsegment und 25 % Hartsegment.

## Beispiel 21:

In einem 2 l Schliffbecherglas werden 900g (0,45 Mol) Desmophen 2020 geschmolzen und unter Rühren 30 Minuten bei 120°C und 15 mbar entwässert. Nach Zugabe von 176,9 g (0,675 Mol) Desmodur W wird unter N₂ auf 150°C erhitzt. Nach 1 Stunde Rühren bei dieser Temperatur beträgt der NCO-Gehalt 1,78 % (berechnet 1.76 %). Dem Prepolymer werden weitere 264,1g (1,01 Mol) Desmodur W und 140 g (1,19 Mol) Hexandiol-1,6 zugesetzt. Anschließend wird der gesamte Ansatz auf 230°C erhitzt und 30 Min. bei dieser Temperatur nachgerührt. Die hochviskose, rührbare Schmelze wird auf ein Teflonblech gegossen. Man erhält ein klares, klebfreies Polyurethan.
Kennzahl: 103
40 Gew.% Hartsegment
60 Gew.% Weichsegment

**Beispiel 22:**

In einem 2 1 Schliffbecherglas werden 800 g (0,8 Mol) Terathane 1000 geschmolzen und unter Rühren 30 Minuten bei 120°C und 20 mbar entwässert. Nach Zugabe von 945 g (3,6 Mol) Desmodur W wird unter N₂ auf 150°C erhitzt. Nach 1 Stunde Rühren bei dieser Temperatur beträgt der NCO-Gehalt 13,8 % (berechnet 13,5 %). Danach tropft man bei 150-200°C 243 g (2,7 Mol) Butandiol-1,4 zu und rührt 1 Stunde bei 200-210°C nach. Die hochviskose, gut rührbare Schmelze wird auf ein Tetlonblech gegossen. Man erhält ein bei Raumtemperatur klebfreies, klares, farbloses Polyurethan mit einem NCO-Gehalt von 0,46 % (berechnet 0,42 %).
Kennzahl: 103
60 Gew.% Hartsegment
40 Gew.% Weichsegment

**Beispiel 23:**

In einem 2 l Schliffbecherglas werden 800 g (0,8 Mol) Terathane 1000 geschmolzen und unter Rühren 30 Minuten bei 120°C und 20 mbar entwässert. Nach Zugabe von 524 g (2 Mol) Desmodur W wird 1 Stunde unter Rühren auf 150°C erhitzt. Der NCO-Gehalt beträgt 7,7 % (berechnet 7,6 %). In einem Guß werden dem Ansatz 251 g (2,79 Mol) Butandiol zugegeben. Danach erwärmt sich der Kolbeninhalt von 130°C auf 154°C. Anschließend tropft man bei 150-200°C 406g (1,55 Mol) Desmodur W zu, rührt 1 Stunde bei 200-210°C nach und gießt die Schmelze auf ein Teflonblech. Bei Raumtemperatur ist das Produkt klar, farblos und klebfrei.
Kennzahl: 99
60 Gew.-% Hartsegment
40 Gew.-% Weichsegment

**Patentansprüche**

1. Katalysatorfreie thermoplastische, Polyurethane, enthaltend aliphatische und/oder cycloaliphatische Diisocyanate, Macrodiole vorn MG 500 bis 10000 und Kettenverlängerer vom MG 62 bis 400, erhältlich durch Umsetzung von aliphatischen und/oder cycloaliphatischen Diisocyanaten mit einem Macrodiol zu einem NCO-Gruppen aufweisenden Voraddukt, wobei man 1.5 bis 22 Mol Diisocyanat pro Mol Macrodiol einsetzt, und die Kettenverlängerung des Voraddukts mit niedermolekularen aliphatischen und/oder cycloaliphatischen Diolen vornimmt, gegebenenfalls weiteres Diisocyanat -unter Beibehaltung des obengenannten NCO/OH-Verhältnisses - als Kettenverlängerer zuführt, man die Voradduktbildung bei 90-150 °C durchführt und die Kettenverlängerung bei Temperaturen von 90-230°C vornimmt, mit der Maßgabe, daß man die genannten Reaktionsbedingungen mindestens so lange aufrecht erhält, bis eine Schmelzviskosität von mindestens 2500 mPas, gemessen bei 120 °C, erreicht ist und anschließend bei erhöhter Temperatur von 130°C bis 240 °C die Polyaddition zu einem beliebigen Zeitpunkt zu Ende führt.

2. Katalysatorfreie thermoplastische, Polyurethane gemäß Anspruch 1, dadurch gekennzeichnet, daß sie 25-75 Gew.-% Hartsegment und 75-25 Gew.-% Weichsegment besitzen

3. Verfahren zur Herstellung von gegebenenfalls in einem organ. Lösemittel gelösten, katalysatorfreien thermoplastischen Polyurethanen durch Umsetzung von aliphatischen und/oder cycloaliphatischen Diisocyanaten mit einem Macrodiol zu einem NCOGruppen aufweisenden Voraddukt, wobei man 1,5 bis 22Mol Diisocyanat pro Mol Macrodiol einsetzt, und die Kettenverlängerung des Voraddukts mit niedermolekularen aliphatischen und/oder cycloaliphatischen Diolen vornimmt, gegebenenfalls weiteres Diisocyanat -unter Beibehaltung des obengenannten NCO/OH-Verhältnisses - als Kettenverlängerer zuführt, dadurch gekennzeichnet, daß man die Voradduktbildung bei 90-150°C durchführt und die Kettenverlängerung bei Temperaturen von 90-230°C vornimmt, mit der Maßgabe, daß man die genannten Reaktionsbedingungen mindestens so lange aufrecht erhält, bis eine Schmelzviskosität von mindestens 2500 mPas, gemessen bei 120 °C, erreicht ist und anschließend bei erhöhter Temperatur von 130°C bis 240 °C die Polyaddition zu einem beliebigen Zeitpunkt zu Ende führt.

4. Verwendung der Polyurethane gemäß Anspruch 1 oder 2 zur Herstellung von Implantaten insbesondere zur Herstellung von Kathetern und Schläuchen, zur Herstellung von Blutbeuteln, medizinisch angewendeten Folien, Klebstoffen und beliebigen Formkörpern als orthopädische Materialien.

5. Verwendung der Polyurethane gemäß Anspruch 1 oder 2 als Folien, Verbundmaterial, Formkörper, Dichtungsmaterial oder Verpackungsmaterial.

## Claims

1. Catalyst-free, thermoplastic polyurethanes containing aliphatic and/or cycloaliphatic diisocyanates, macrodiols of an MW of 500 to 10000 and chain extenders of an MW of 62 to 400, obtainable by reacting aliphatic and/or cycloaliphatic diisocyanates with a macrodiol to yield a pre-adduct containing NCO groups, wherein 1.5 to 22 mol of diisocyanate are used per mol of macrodiol, and the pre-adduct is chain, extended with low molecular weight aliphatic and/or cycloaliphatic diols, further diisocyanate is optionally added (while maintaining the above-stated NCO/OH ratio) as a chain extender, pre-adduct formation is performed at 90 to 150°C and chain extension is performed at 90 to 230°C, with the proviso that the stated reaction conditions are maintained at least until a melt viscosity of at least 2500 mPa·s, measured at 120°C, is achieved and, at any desired time, polyaddition is then brought to completion at an elevated temperature of 130°C to 240°C.

2. Catalyst-free, thermoplastic polyurethanes according to claim 1, characterised in that they have 25-75 wt.% of hard segment and 75 to 25 wt.% of soft segment.

3. Process for the production of catalyst-free, thermoplastic polyurethanes optionally dissolved in an organic solvent by reacting aliphatic and/or cycloaliphatic diisocyanates with a macrodiol to yield a pre-adduct containing NCO groups, wherein 1.5 to 22 mol of diisocyanate are used per mol of macrodiol and the pre-adduct is chain-extended with low molecular weight aliphatic and/or cycloaliphatic diols, further diisocyanate is optionally added (while maintaining the above-stated NCO/OH ratio) as a chain extender, characterised in that pre-adduct formation is performed at 90 to 150°C and chain extension is performed at 90 to 230°C, with the proviso that the stated reaction conditions are maintained at least until a melt viscosity of at least 2500 mPa·s, measured at 120°C, is achieved and, at any desired time, polyaddition is then brought to completion at an elevated temperature of 130°C to 240°C.

4. Use of the polyurethanes according to claim 1 or 2 for the production of implants, in particular for the production of catheters and tubes, for the production of blood pouches, films, adhesives for medical applications and any desired mouldings as orthopaedic materials.

5. Use of the polyurethanes according to claim 1 or 2 as films, composite material, mouldings, sealing material or packaging material.

## Revendications

1. Polyuréthanes thermoplastiques exempts de catalyseurs, contenant des diisocyanates aliphatiques et/ou cycloaliphatiques, des macrodiols de PM 500 à 10 000 et des agents d'allongement des chaînes de PM 62 à 400, qu'on obtient en faisant réagir des diisocyanates aliphatiques et/ou cycloaliphatiques avec un macrodiol, la réaction donnant un préadduct à groupes NCO, à raison de 1,5 à 22 mol de diisocyanate par mol du macrodiol, et on procède à l'allongement des chaînes du préadduct à l'aide de diols aliphatiques et/ou cycloaliphatiques à bas poids moléculaire, le cas échéant d'un complément de diisocyanate -en maintenant le rapport NCO/OH spécifié- en tant qu'agents d'allongement des chaînes, on procède à la formation du préadduct à des températures de 90 à 150°C, et à l'allongement des chaînes à des températures de 90 à 230°C, étant spécifié que l'on maintient les conditions de réaction indiquées au moins jusqu'à ce que la viscosité à l'état fondu soit d'au moins 2 500 mPas, la mesure étant faite à 120°C, après quoi on termine la polyaddition à un moment quelconque, à une température plus élevée, allant de 130°C à 240°C.

2. Polyuréthanes thermoplastiques exempts de catalyseurs selon la revendication 1, caractérisés en ce qu'ils possèdent 25 à 75 % en poids de segments durs et 75 à 25 % en poids de segments mous.

3. Procédé pour la préparation de polyuréthanes thermoplastiques exempts de catalyseurs, éventuellement à l'état de solution dans un solvant organique, par réaction de diisocyanates aliphatiques et/ou cycloaliphatiques avec un macrodiol, donnant un préadduct à groupes NCO, à raison de 1,5 à 22 mol de diisocyanate par mol du macrodiol, et allongement des chaînes du préadduct à l'aide de diols aliphatiques et/ou cycloaliphatiques à bas poids moléculaire, le cas échéant d'un complément de diisocyanate -en maintenant le rapport NCO/OH spécifié ci-dessus- en tant qu'agents d'allongement des chaînes, caractérisé en ce que l'on procède à la formation du préadduct à des températures de 90 à 150°C, et à l'allongement des chaînes à des températures de 90 à 230°C, étant spécifié que ces conditions de réaction sont maintenues au moins jusqu'à ce que la viscosité à l'état fondu soit d'au moins 2 500 mPas, la mesure étant faite à 120°C, après quoi on termine la polyaddition à un moment quelconque, à une température plus élevée, allant de 130°C à 240°C.

4. Utilisation des polyuréthanes selon la revendication 1 ou 2 pour la fabrication d'implants et en particulier pour la fabrication de cathéters et de tuyaux souples, pour la fabrication de sachets à sang, de feuilles et colles utilisables en médecine et d'objets moulés quelconques en tant que matériaux orthopédiques.

5. Utilisation des polyuréthanes selon la revendication 1 ou 2, à l'état de feuilles, de matière composite, d'objets moulés, de matière d'étanchéité ou de matière d'emballage.